# EUROPEAN PATENT APPLICATION

(11) **EP 3 739 494 A2**
(43) Date of publication of application: **18.11.2020**
(21) Application number: 20171452.4
(22) Date of filing: 27.04.2020
(51) Int. Cl.: G06F 30/23, G06F 30/27, G16C 10/00, G16C 60/00, H01M 6/18

(54) **METHOD, APPARATUS, SYSTEM, AND PROGRAM FOR OPTIMIZING SOLID ELECTROLYTES FOR LI-ION BATTERIES USING BAYESIAN OPTIMIZATION**

(30) Priority: 16.05.2019 JP 2019093052
(71) Applicant: FUJITSU LIMITED, Kanagawa 211-8588 (JP)
(72) Inventor: LIU, Yu, Kawasaki-shi, Kanagawa 211-8588 (JP)
(74) Representative: Haseltine Lake Kempner LLP

(57) **Abstract**

An optimization apparatus, includes: a control unit configured to generate, on the basis of input data, a Bayesian model obtained by modeling a problem of searching for a combination of values of a plurality of parameters which gives an optimum value of a characteristic value regarding a target substance, generate, on the basis of one or several input values of parameters among the plurality of parameters, a search space that is included in an entire search space obtained from all combinations of the values of the plurality of parameters and is narrower than the entire search space, and execute a search for the combination using the Bayesian model in the search space.

## Description

### FIELD

The embodiments discussed herein are related to an optimization apparatus, an optimization system, an optimization method, and an optimization program.

### BACKGROUND

In recent years, a field called materials informatics has attracted attention, which is for improving quality and efficiency of material design/manufacturing by adding an information processing technology using artificial intelligence (AI) or the like to a material design/manufacturing process. Materials informatics uses data obtained from experiments or theoretical calculations to search for characteristic factors or the like of new materials on the basis of statistical learning.

As an example of materials informatics, Bayesian inference is combined with first-principle calculation that is one of material simulation methods to determine an optimal composition of a high-performance material.

Bayesian inference is a method of inferring an optimal event from observed events by a statistical approach on the basis of Bayesian probability. In the above example, the number of calculations of the first-principle calculation until the optimal composition is obtained can be reduced by applying Bayesian inference.

A tool for performing optimization using Bayesian inference is, for example, COMmon Bayesian Optimization (COMBO).

As a related art, for example, there has been proposed a technology of calculating a posterior probability from a possibility of an ionic radius value and a prior probability on the basis of Bayesian theory to obtain a radius value distribution regarding an arbitrary oxidation number of a target element. Further, there has also been proposed a technology of performing machine learning on substance models modeled on the basis of known substances, inputting a target physical property to the learning result to determine candidate substances, and determining a new substance from the candidate substances.

Japanese Laid-open Patent Publication No. 2015-109084 and Japanese Laid-open Patent Publication No. 2017-91526 are disclosed as related art.

In a process of searching for a combination of values of a plurality of parameters (hereinafter, referred to as "optimal solution") which gives an optimum value of a characteristic value regarding a target substance by Bayesian inference, a prior distribution of a Bayesian model is first determined, and then the above combination of the values of the parameters is searched for on the basis of the prior distribution. For example, experimental data, simulation data, and the like are used as input data in a case where the prior distribution is set.

In Bayesian inference, a search result changes depending on the prior distribution. However, when the prior distribution is set in a situation where the input data is limited and Bayesian inference is performed, a search result (solution) is likely to deviate from an optimal solution. Thus, it is difficult to accurately search for the optimal solution.

In an aspect, an object of the embodiments is to provide an optimization apparatus, an optimization system, an optimization method, and an optimization program for accurately searching for an optimal solution even in a case where input data is limited.

### SUMMARY

According to an aspect of the embodiments, an optimization apparatus, includes: a control unit configured to generate, on the basis of input data, a Bayesian model obtained by modeling a problem of searching for a combination of values of a plurality of parameters which gives an optimum value of a characteristic value regarding a target substance, generate, on the basis of one or several input values of parameters among the plurality of parameters, a search space that is included in an entire search space obtained from all combinations of the values of the plurality of parameters and is narrower than the entire search space, and execute a search for the combination using the Bayesian model in the search space.

According to an aspect, it is possible to accurately search for an optimal solution even in a case where input data is limited.

### BRIEF DESCRIPTION OF DRAWINGS

Embodiments are set out, by way of example only, with reference to the following drawings, in which:
FIG. 1 illustrates an example of an optimization system according to a first embodiment;
FIG. 2 is an explanatory diagram of an example of generating a Bayesian model (part 1);
FIG. 3 is an explanatory diagram of an example of generating a Bayesian model (part 2);
FIG. 4 illustrates an example of updating a Bayesian model;
FIG. 5 illustrates an example of hardware of an optimization apparatus according to a second embodiment;
FIG. 6 illustrates an example of functional blocks of an optimization apparatus;
FIG. 7 illustrates an example of a slider space;
FIG. 8 is a flowchart illustrating an example of operation for generating a slider space;
FIG. 9 is a flowchart illustrating an example of operation of an optimization apparatus; and
FIG. 10 illustrates an example of results of comparison between a search using the entire search space and a search using a slider space.

### DESCRIPTION OF EMBODIMENTS

Hereinafter, embodiments will be described with reference to the drawings.

### [First embodiment]

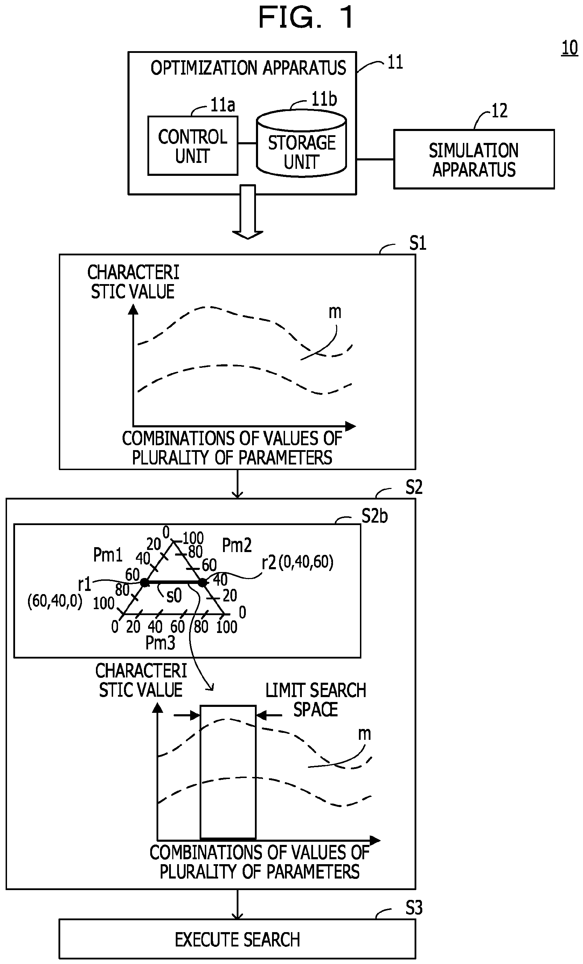

FIG. 1 illustrates an example of an optimization system according to a first embodiment.

An optimization system 10 according to the first embodiment includes an optimization apparatus 11 and a simulation apparatus 12.

The optimization apparatus 11 includes a control unit 11a and a storage unit 11b.

The control unit 11a generates, on the basis of input data, a Bayesian model obtained by modeling a problem of searching for a combination (optimal solution) of values of a plurality of parameters which gives an optimum value of a characteristic value regarding a target substance.

The input data is simulation results, experimental results, or the like of characteristic values obtained when several combinations of the values of the plurality of parameters are used. As the characteristic value regarding the target substance, various values are applicable, such as conductivity and a dielectric constant of the target substance. The plurality of parameters may be, for example, a mixing ratio of a plurality of materials contained in the target substance. Further, the plurality of parameters regarding the target substance may be, for example, various manufacturing conditions set when the target substance is manufactured, such as a pressure and a temperature. An example of generating a Bayesian model will be described below.

Further, the control unit 11a generates, on the basis of one or several input values of parameters among the plurality of parameters, a search space that is included in the entire search space obtained from all combinations of the values of the plurality of parameters and is narrower than the entire search space. Then, the control unit 11a executes a search for an optimal solution using the generated Bayesian model in the determined search space.

As the one or several values of the parameters, it is possible to use values expected to have a sufficient characteristic value (such as a know-how value that is considered to be appropriate on the basis of experience that a user has had so far). Alternatively, the one or several values of the parameters may be a value (s) of a parameter(s) based on a constraint for obtaining a sufficient characteristic value. For example, in a case where a sufficient characteristic value is not obtained unless a temperature falls within a predetermined range, the temperature within the range is applicable as the one or several values of the parameters.

In the search process, the control unit 11a searches for a combination of values of parameters which maximizes a value of a function called acquisition function that is obtained on the basis of the Bayesian model and information regarding the search space under a certain strategy. It is possible to use strategies such as probability of improvement (PI), expected improvement (EI), and Thompson sampling (TS).

The control unit 11a transmits the combination of the values of the plurality of parameters obtained by the above search to the simulation apparatus 12. Note that the control unit 11a may display the obtained combination of the values of the plurality of parameters on a display (not illustrated).

The control unit 11a is a processor such as a central processing unit (CPU) or a digital signal processor (DSP). However, the control unit 11a may include an electronic circuit for a special use, such as an application specific integrated circuit (ASIC) or a field programmable gate array (FPGA). The processor executes programs stored in a memory such as a random access memory (RAM). For example, an optimization program is executed. Note that a set of a plurality of processors may be referred to as "multiprocessor" or simply as "processor".

The storage unit 11b stores, for example, the input data from the simulation apparatus 12, the one or several values of the parameters described above, and the like.

The storage unit 11b is a volatile storage device such as a RAM, or a non-volatile storage device such as a hard disk drive (HDD) or a flash memory.

The simulation apparatus 12 calculates a characteristic value on the basis of the combination of the values of the plurality of parameters supplied as a search result from the optimization apparatus 11. Then, the simulation apparatus 12 transmits, to the optimization apparatus 11, the characteristic value and the combination of the values of the plurality of parameters indicating the characteristic value.

Next, operation (optimization method) of the optimization apparatus 11 will be described by using the example illustrated in FIG. 1.
[Step S1] The control unit 11a generates a Bayesian model on the basis of input data. FIG. 1 illustrates an example of a Bayesian model m. The horizontal axis indicates a combination of values of a plurality of parameters, and the vertical axis indicates a characteristic value. The Bayesian model m has an uncertainty region defined by variance described below in the vertical axis direction. An example of generating a Bayesian model will be described below.

[Step S2] The control unit 11a generates a search space for the Bayesian model on the basis of one or several input values of parameters among the plurality of parameters. The example of FIG. 1 illustrates, as the plurality of parameters, a mixing ratio of Pm1, Pm2, and Pm3 used in a case where three materials are mixed to obtain a target substance. Further, in the example of FIG. 1, a linear space s0 connecting a point r1 where Pm1 = 60%, Pm2 = 40%, and Pm3 = 0% are satisfied and a point r2 where Pm1 = 0%, Pm2 = 40%, and Pm3 = 60% are satisfied is generated as the search space.

[Step S3] In the determined search space, the control unit 11a executes a search for a combination of the values of the plurality of parameters which gives an optimum value of a characteristic value by using the Bayesian model.

Note that, although not illustrated, the control unit 11a transmits the combination of the values of the plurality of parameters obtained by the above search to the simulation apparatus 12 thereafter. The simulation apparatus 12 calculates a characteristic value on the basis of the combination of the values of the plurality of parameters supplied from the optimization apparatus 11, and transmits, to the optimization apparatus 11, the characteristic value and the combination of the values of the plurality of parameters from which the characteristic value is obtained. The control unit 11a of the optimization apparatus 11 updates the Bayesian model m on the basis of the received information. An example of updating the Bayesian model m will be described below.

As described above, in a case where the optimization apparatus 11 searches for a combination of values of a plurality of parameters which gives an optimum value of a characteristic value of a target substance by Bayesian inference, the optimization apparatus 11 limits a search space on the basis of one or several input values of parameters. In a case where a search is performed in the entire search space, accuracy of the generated Bayesian model is poor in a situation where the input data is limited, and a search result (solution) is likely to deviate from the optimal solution. Thus, it is difficult to accurately search for the optimal solution. On the other hand, when a search range is limited on the basis of the one or several input values of the parameters, it is possible to give a direction of search so as to obtain a sufficient search result, and it is also possible to reflect know-how or the like. Thus, it is possible to accurately search for the optimal solution even in a case where the input data is limited.

Further, the control unit 11a updates the Bayesian model on the basis of the combination of the values of the plurality of parameters obtained by the above search and the characteristic value based on the combination calculated by the simulation apparatus 12, thereby improving the accuracy of the Bayesian model. By repeating such an updating process, the optimal solution can be searched for more accurately.

Note that, for example, the user may obtain the characteristic value by an experiment or the like on the basis of the combination of the values of the plurality of parameters obtained by the search performed by the optimization apparatus 11. Then, the user may input, as new input data, the characteristic value obtained from the experiment or the like and the combination of the values of the plurality of parameters from which the characteristic value is obtained to the optimization apparatus 11 by using an input device or the like. In that case, the simulation apparatus 12 need not be provided.

### <Examples of generating and updating Bayesian model>

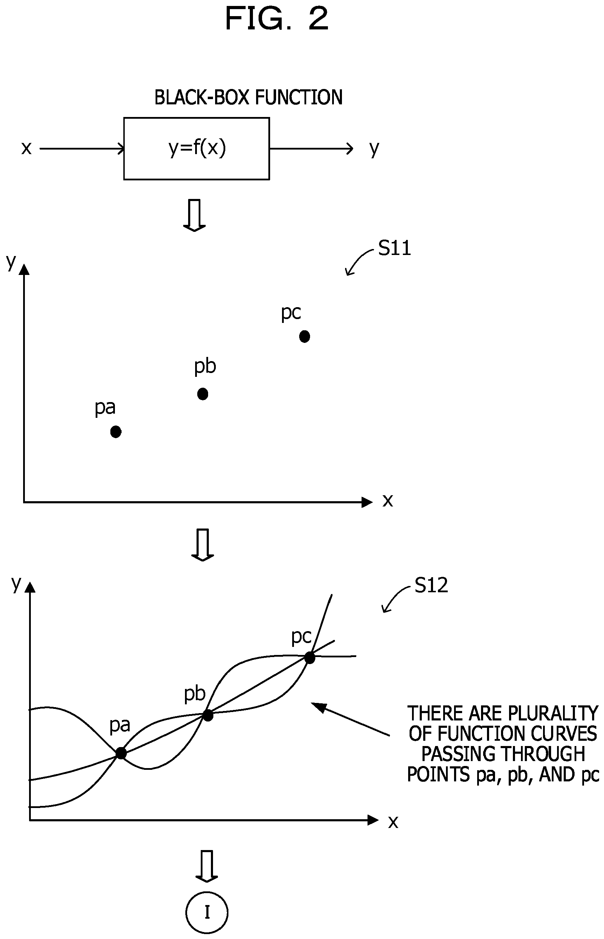

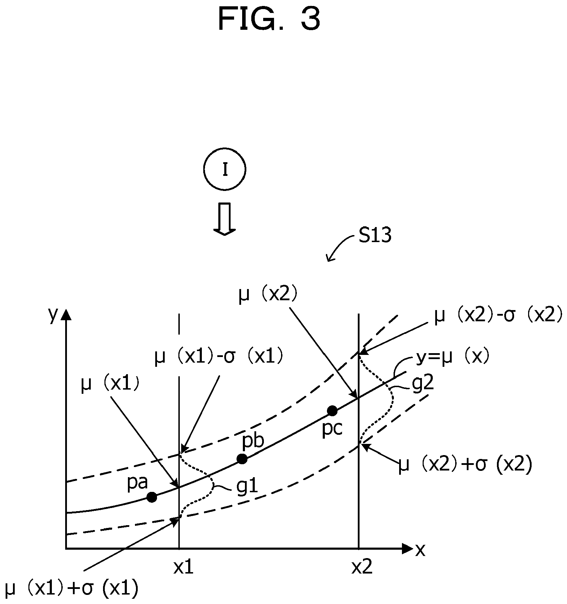

Next, examples of generating and updating a Bayesian model will be described with reference to FIGs. 2 to 4. FIGs. 2 and 3 are explanatory diagrams of an example of generating a Bayesian model. In this example, there is a black-box function in which y is output when x is input, and the Bayesian model has higher accuracy as a shape thereof is closer to this function. Note that the horizontal axis x in FIGs. 2 and 3 indicates an input value of the function, and the vertical axis y therein indicates an output value of the function. The input value of the function corresponds to the combination of the values of the plurality of parameters described above, and the output value of the function corresponds to the characteristic value based on the combination described above.

[Step S11] Three points pa, pb, and pc are observed by an experiment or simulation. For example, those points correspond to input data for generating a first Bayesian model.

[Step S12] Although a curve of the black-box function certainly passes through the points pa, pb, and pc, it may be impossible to determine what kind of function curve will be obtained at this stage (There are a plurality of curves passing through the points pa, pb, and pc.). In Bayesian inference, modeling (Bayesian modeling) is performed by applying a Gaussian process to such an uncertainty part and inferring an average function and variance at x.

[Step S13] The average function passing through the points pa, pb, and pc is set to y = µ(x). In a case where x1 is observed, variance of y = µ(x1) is calculated as σ(x1). In this case, a Gaussian distribution g1 illustrated in FIG. 3 is applied to y = µ(x1).

Similarly, variance of y = µ(x2) is calculated as µ(x2), and a Gaussian distribution g2 is applied to y = µ(x2). By applying the Gaussian process in this way, the uncertainty part can be formulated by the variance.

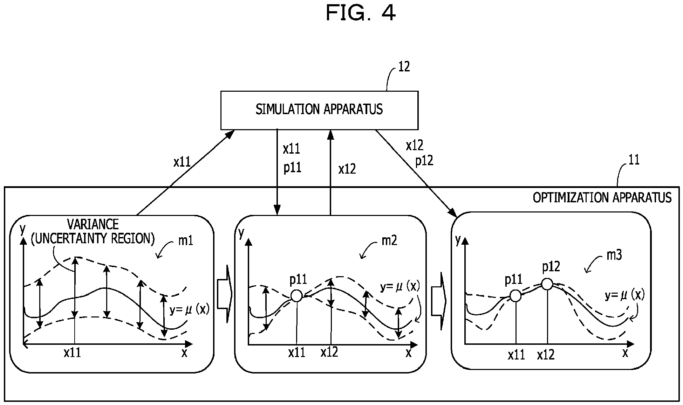

FIG. 4 illustrates an example of updating a Bayesian model.

In a case where x11 is obtained as a search result by a search using a Bayesian model m1 generated on the basis of input data (initial data), the optimization apparatus 11 transmits x11 to the simulation apparatus 12.

The simulation apparatus 12 calculates a characteristic value p11 on the basis of x11 supplied from the optimization apparatus 11, and transmits p11 and x11 to the optimization apparatus 11. The optimization apparatus 11 generates a Bayesian model m2 by updating the Bayesian model m1 on the basis of p11 and x11.

Then, in a case where x12 is obtained as a search result by a search using the Bayesian model m2, the optimization apparatus 11 transmits x12 to the simulation apparatus 12.

The simulation apparatus 12 calculates a characteristic value p12 on the basis of x12 supplied from the optimization apparatus 11, and transmits p12 and x12 to the optimization apparatus 11. The optimization apparatus 11 generates a Bayesian model m3 by updating the Bayesian model m2 on the basis of p12 and x12.

Repeating such operation gradually reduces the uncertainty region. This makes it possible to generate an accurate Bayesian model closer to the black-box function.

### [Second embodiment]

Next, a second embodiment will be described.

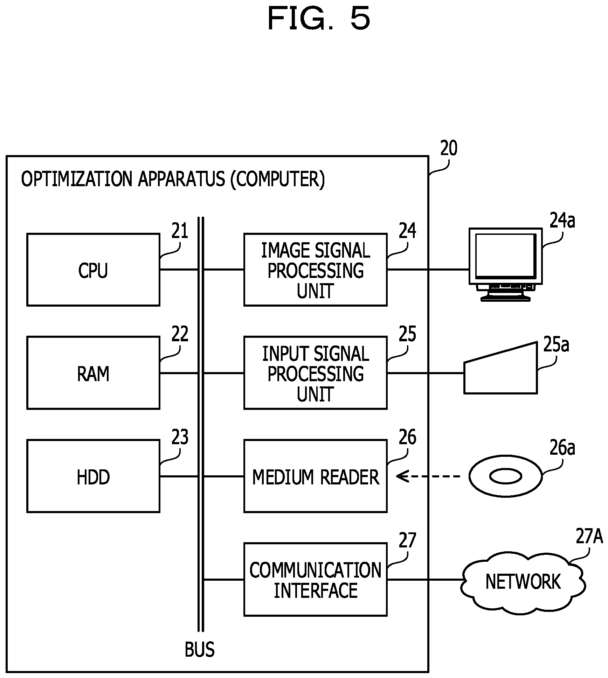

FIG. 5 illustrates an example of hardware of an optimization apparatus according to the second embodiment.

An optimization apparatus 20 according to the second embodiment is, for example, a computer, and includes a CPU 21, a RAM 22, an HDD 23, an image signal processing unit 24, an input signal processing unit 25, a medium reader 26, and a communication interface 27. The above units are connected to a bus.

The CPU 21 is a processor including an arithmetic circuit that executes a command of a program. The CPU 21 loads at least a part of the programs and data stored in the HDD 23 into the RAM 22, and executes the programs. Note that the CPU 21 may include a plurality of processor cores, and the optimization apparatus 20 may include a plurality of processors. Processing described below may be executed in parallel by using the plurality of processors or processor cores. Further, a set of a plurality of processors (multiprocessor) may also be referred to as "processor".

The RAM 22 is a volatile semiconductor memory that temporarily stores the programs executed by the CPU 21 and the data used for calculation by the CPU 21. Note that the optimization apparatus 20 may include a different kind of memory other than the RAM, or may include a plurality of memories.

The HDD 23 is a non-volatile storage device that stores programs and data of software such as an operating system (OS), middleware, and application software. The programs include, for example, a control program of the optimization apparatus 20. Note that the optimization apparatus 20 may include a different kind of storage device such as a flash memory or a solid state drive (SSD), or may include a plurality of non-volatile storage devices.

The image signal processing unit 24 outputs an image to a display 24a connected to the optimization apparatus 20 in response to a command from the CPU 21. The display 24a can be a cathode ray tube (CRT) display, a liquid crystal display (LCD), a plasma display (plasma display panel: PDP), an organic electro-luminescence (organic EL: OEL) display, or the like.

The input signal processing unit 25 acquires an input signal from an input device 25a connected to the optimization apparatus 20, and outputs the input signal to the CPU 21. The input device 25a can be a pointing device such as a mouse, a touch panel, a touchpad, or a trackball, a keyboard, a remote controller, a button switch, or the like. Further, a plurality of kinds of input devices may be connected to the optimization apparatus 20.

The medium reader 26 is a reading device that reads programs and data recorded on the recording medium 26a. The recording medium 26a can be, for example, a magnetic disk, an optical disk, a magneto-optical (MO) disk, a semiconductor memory, or the like. The magnetic disk encompasses a flexible disk (FD) and an HDD. The optical disk encompasses a compact disc (CD) and a digital versatile disc (DVD).

The medium reader 26 copies, for example, a program or data read from the recording medium 26a to another recording medium such as the RAM 22 or the HDD 23. The read program is executed by, for example, the CPU 21. Note that the recording medium 26a may be a portable recording medium, and may be used for distributing the programs and data. Further, the recording medium 26a and the HDD 23 may be referred to as "computer-readable recording media".

The communication interface 27 is an interface that is connected to a network 27a and communicates with another information processing apparatus via the network 27a. The communication interface 27 may be a wired communication interface connected to a communication device such as a switch by a cable, or a wireless communication interface connected to a base station via a wireless link.

Note that the optimization apparatus 11 and the simulation apparatus 12 illustrated in FIG. 1 can also be realized by the hardware illustrated in FIG. 5.

### <Functional blocks>

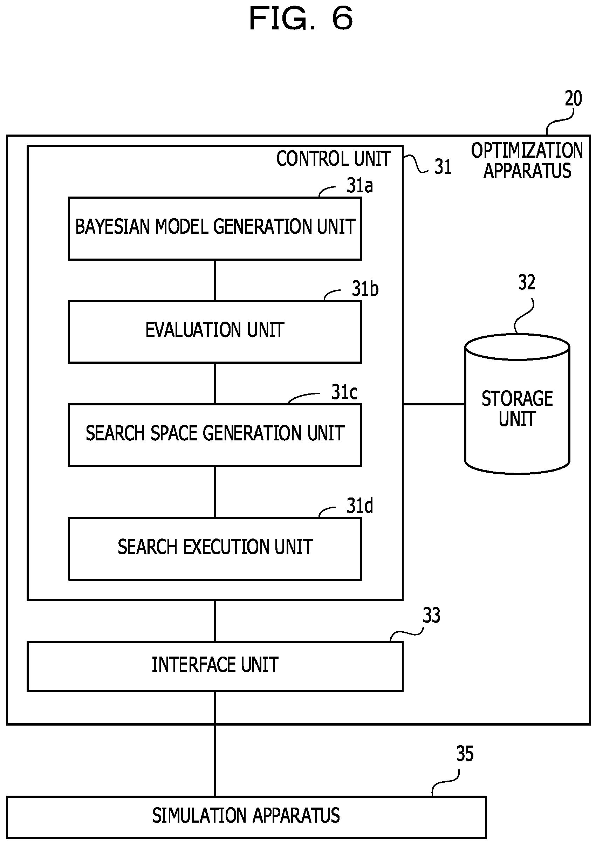

FIG. 6 illustrates an example of functional blocks of the optimization apparatus.

The optimization apparatus 20 includes a control unit 31, a storage unit 32, and an interface unit 33. The control unit 31 includes a Bayesian model generation unit 31a, an evaluation unit 31b, a search space generation unit 31c, and a search execution unit 31d.

The Bayesian model generation unit 31a generates, on the basis of input data, a Bayesian model obtained by modeling a problem of searching for a combination of values of a plurality of parameters which gives an optimum value of a characteristic value regarding a target substance.

The evaluation unit 31b evaluates accuracy of the Bayesian model on the basis of a result of comparison between an accuracy evaluation value indicating the accuracy of the Bayesian model and a threshold. The accuracy evaluation value is, for example, the variance of the Bayesian model illustrated in FIG. 4 and the like.

In a case where it is determined that the accuracy evaluation value is equal to or more than the threshold (in a case where the accuracy is evaluated to be poor), the search space generation unit 31c generates a search space on the basis of one or several input values of parameters. Hereinafter, this search space will be referred to as "slider space". The slider space corresponds to, for example, the linear space s0 illustrated in FIG. 1. In a case where it is determined that the accuracy evaluation value is less than the threshold (in a case where the accuracy is evaluated to be sufficient), the search space generation unit 31c determines all combinations of the values of all the parameters (entire search space) as the search space.

The search execution unit 31d executes a search for an optimal solution that gives an optimum value of a characteristic value in the obtained search space.

The storage unit 32 stores, for example, input data from a simulation apparatus 35, the one or several input values of the parameters described above, and the like.

The interface unit 33 transmits and receives data between the control unit 31 and the outside of the optimization apparatus 20 (e.g., the simulation apparatus 35).

Note that the control unit 31 can be implemented by using, for example, a program module executed by the CPU 21. The storage unit 32 can be implemented by using, for example, a storage area secured in the RAM 22 or the HDD 23. The interface unit 33 can be implemented by using, for example, the input signal processing unit 25 and the communication interface 27 in FIG. 5. Note that the simulation apparatus 35 can be realized by the hardware illustrated in FIG. 5.

### <Slider space>

Next, an example of the slider space will be described. Note that, hereinafter, a case of searching for a mixing ratio that gives an optimum value of ionic conductivity regarding an electrolyte of a lithium ion battery synthesized from three materials (lithium-containing oxoates) will be described as an example.

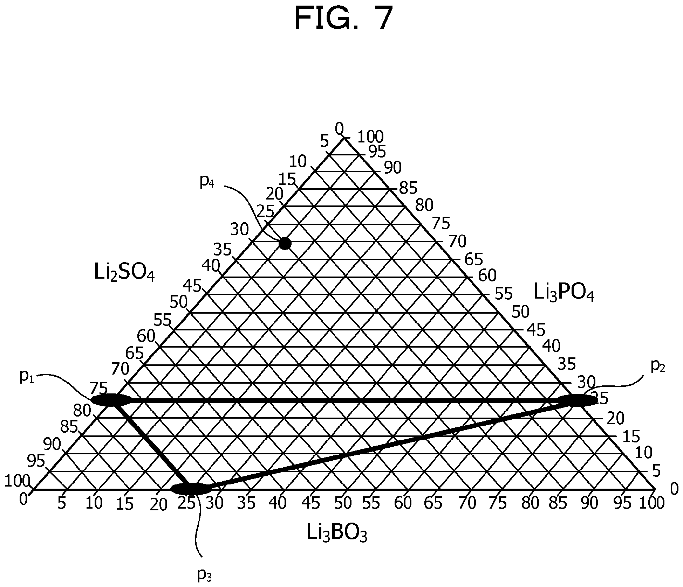

FIG. 7 illustrates an example of the slider space.

FIG. 7 illustrates mixing ratios of lithium sulfate (Li₂SO₄), lithium phosphate (Li₃PO₄), and lithium borate (Li₃BO₃) serving as the three lithium-containing oxoates. Points p₁, p₂, and p₃ are obtained from, for example, experiments, know-how, or the like, and are represented by using mixing ratios of two materials having high ionic conductivity. Information regarding the mixing ratios for representing those points is input from, for example, the outside. Further, in a case where the information regarding the mixing ratios for representing those points, which is input from the outside, is stored in the storage unit 32 in advance, the control unit 31 may use the information by reading the information from the storage unit 32.

The point p₁ is a point where the mixing ratio of Li₂SO₄, Li₃PO₄, and Li₃BO₃ is 75%, 25%, and 0%. The point p₂ is a point where the mixing ratio of Li₂SO₄, Li₃PO₄, and Li₃BO₃ is 0%, 25%, and 75%. The point p₃ is a point where the mixing ratio of Li₂SO₄, Li₃PO₄, and Li₃BO₃ is 75%, 0%, and 25%.

The slider space is a linear space connecting those points p₁ to p₃ in the example of FIG. 7. Note that a point p₄ will be described below.

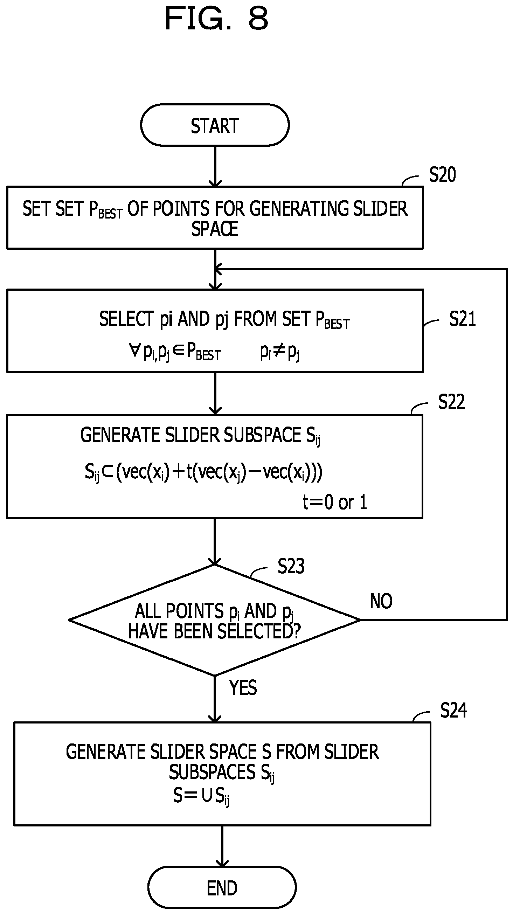

FIG. 8 is a flowchart illustrating an example of operation for generating a slider space.

[Step S20] The search space generation unit 31c sets a set P_{best} of points for generating a slider space on the basis of one or several input values of parameters. The number of sets P_{best} is 2 or more. In the example of FIG. 7, the set P_{best} is three points p₁ to p₃.

[Step S21] The search space generation unit 31c selects two arbitrary points pᵢ and pⱼ from the set P_{best}. Note that all the points pᵢ and pⱼ belong to the set P_{best}, and the points pᵢ and pⱼ are different from each other.

[Step S22] The search space generation unit 31c generates a slider subspace Sᵢⱼ. The slider subspace Sᵢⱼ can be represented by Sᵢⱼ ⊂ (vec(xᵢ) + t(vec(xⱼ) - vec(xᵢ))). The character "vec(xᵢ)" represents a design parameter for the point pᵢ, and the character "vec(xⱼ)" represents a design parameter for the point pⱼ. The character "t" is 0 or 1. In the example of FIG. 7, the design parameter vec(x₁) of the point p₁ is vec(x₁) = (75, 25, 0).

In the example of FIG. 7, in a case where the points p₁ and p₂ are selected in the processing of step S21, a linear slider subspace S₁₂ connecting the points p₁ and p₂ is generated, and, in a case where the points p₂ and p₃ are selected, a linear slider subspace S₂₃ connecting the points p₂ and p₃ is generated. Further, in a case where the points p₁ and p₃ are selected in the processing of step S21, a linear slider subspace S₁₃ connecting the points p₁ and p₃ is generated.

[Step S23] The search space generation unit 31c determines whether or not all the points pᵢ and pⱼ have been selected from the set P_{best}. In a case where not all the points pᵢ and pⱼ have been selected from the set P_{best}, the search space generation unit 31c repeats the processing from step S21.

[Step S24] In a case where it is determined that all the points pᵢ and pⱼ have been selected from the set P_{best}, the search space generation unit 31c obtains the union of the slider subspaces Sᵢⱼ and generates a slider space S (Lines of a plurality of slider subspaces are gathered to form lines of a slider space.).

### <Operation flowchart>

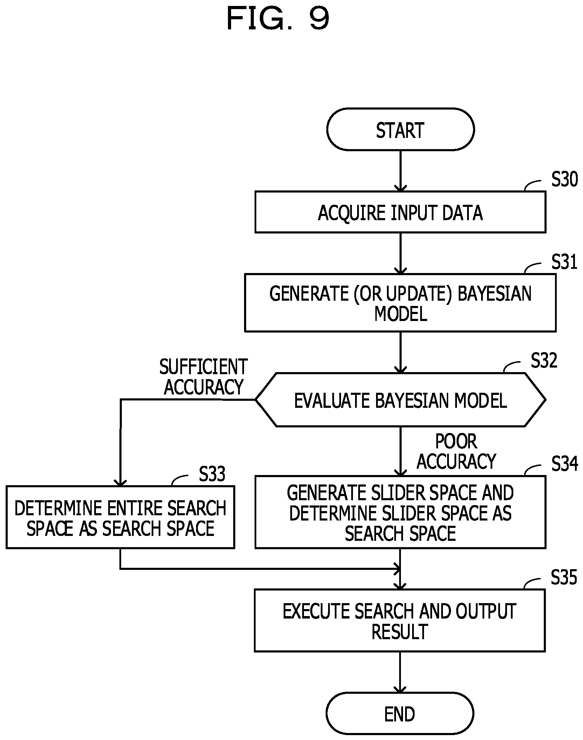

FIG. 9 is a flowchart illustrating an example of operation of the optimization apparatus.

[Step S30] The interface unit 33 acquires input data. The input data includes, for example, simulation results or experimental results of ionic conductivity obtained when several combinations of the mixing ratios of the above-described three materials are used, and one or several values of the parameters for setting the above-described set P_{best}.

[Step S31] The Bayesian model generation unit 31a generates (or updates) a Bayesian model on the basis of the acquired input data.

[Step S32] The evaluation unit 31b evaluates whether or not accuracy of the Bayesian model is sufficient on the basis of a result of comparison between an accuracy evaluation value indicating the accuracy of the Bayesian model (e.g., the variance of the Bayesian model illustrated in FIG. 4) and a threshold. For example, in a case where the variance of the Bayesian model is equal to or more than the threshold, the evaluation unit 31b determines that the accuracy is poor, and, in a case where the variance of the Bayesian model is less than the threshold, the evaluation unit 31b determines that the accuracy is sufficient.

[Step S33] In a case where the search space generation unit 31c determines that the accuracy of the Bayesian model is sufficient, the search space generation unit 31c determines all combinations of values of all parameters (entire search space) as a search space.
[Step S34] In a case where the search space generation unit 31c determines that the accuracy of the Bayesian model is poor, the search space generation unit 31c generates a slider space by the processing illustrated in FIG. 8, and determines the slider space as the search space.

[Step S35] The search execution unit 31d executes a search for a combination (optimal solution) that gives an optimum value of a characteristic value among the combinations included in the obtained search space by using the Bayesian model and causes the interface unit 33 to output the search result.

Thus, one optimization operation of the optimization apparatus 20 is completed. The simulation apparatus 35 calculates a characteristic value (ionic conductivity in the above-described example) on the basis of the search result supplied from the optimization apparatus 20, and transmits the search result and the characteristic value as new input data to the optimization apparatus 20. The optimization apparatus 20 updates the Bayesian model on the basis of the new input data, and repeats the processing from step S32. Note that the optimization apparatus 20 may update the set P_{best} on the basis of the new input data. In that case, the slider space is also updated.

In a case where the search is performed by using the entire search space and the accuracy of the Bayesian model is poor, an erroneous search result illustrating that a combination having the highest ionic conductivity is a combination deviating from the optimal solution (e.g., the point p₄ in FIG. 7) may be obtained.

On the other hand, by limiting a search range to the slider space generated on the basis of the one or several input values of the parameters, the optimization apparatus 20 can give a direction of search so as to obtain a sufficient search result, and can also reflect know-how or the like. Thus, it is possible to accurately search for the optimal solution even in a case where the input data is limited.

Further, the Bayesian model generation unit 31a updates the Bayesian model on the basis of the combination of the values of the plurality of parameters obtained by the above search and the characteristic value based on the combination calculated by the simulation apparatus 35, thereby improving the accuracy of the Bayesian model. By repeating such an updating process, the optimal solution can be searched for more accurately.

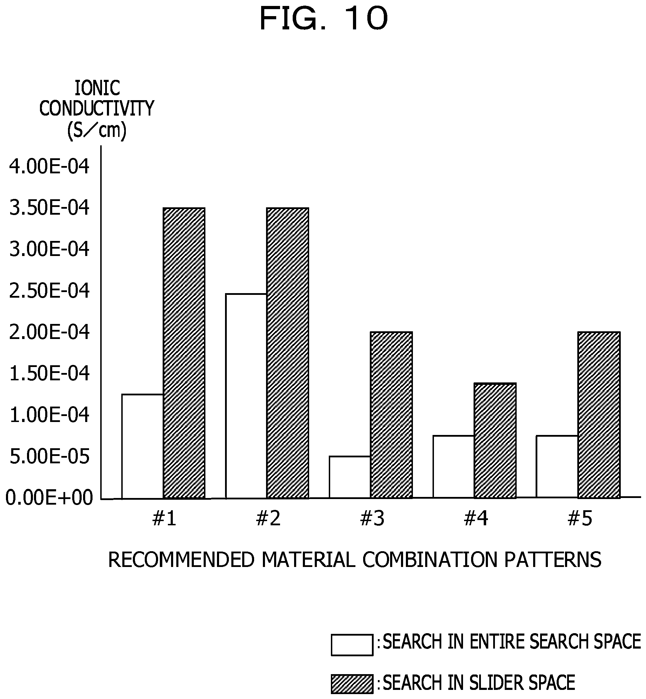

FIG. 10 illustrates an example of results of comparison between a search using the entire search space and a search using a slider space.
FIG. 10 illustrates measurement results of ionic conductivity corresponding to search results of five combination patterns of materials (e.g., there is a combination of the above-described three materials as one pattern) serving as candidate materials for an electrolyte of a lithium ion battery. The number of combinations of the mixing ratios of the above-described three materials included in the initial input data is ten.

The electrolyte was synthesized on the basis of a search result obtained from the search using the entire search space or the search using the slider space, and ionic conductivity of the synthesized electrolyte was measured. As a result, higher ionic conductivity is obtained by using the slider space in any pattern.

By the way, as described above, content of the processing described above can be realized by causing the optimization apparatus 20 to execute programs.

The programs can be recorded on a computer-readable recording medium (e.g., the recording medium 26a). The recording medium can be, for example, a magnetic disk, an optical disk, a magneto-optical disk, a semiconductor memory, or the like. The magnetic disks encompass an FD and an HDD. The optical disk encompasses a CD, a CD-R (recordable)/RW (rewritable), a DVD, and a DVD-R/RW. The programs may be recorded on a portable recording medium to be distributed. In that case, the programs may be copied from the portable recording medium to another recording medium (e.g., the HDD 23) to be executed.

Hereinabove, the embodiments have been described. However, the configuration of each unit described in the embodiments can be replaced with another configuration having a similar function. Further, other arbitrary components and steps may be added. Furthermore, two or more arbitrary configurations (features) in the above-described embodiments may be combined.

## Claims

1. An optimization apparatus, comprising
a control unit configured to generate, on the basis of input data, a Bayesian model obtained by modeling a problem of searching for a combination of values of a plurality of parameters which gives an optimum value of a characteristic value regarding a target substance, generate, on the basis of one or several input values of parameters among the plurality of parameters, a search space that is included in an entire search space obtained from all combinations of the values of the plurality of parameters and is narrower than the entire search space, and execute a search for the combination using the Bayesian model in the search space.

2. The optimization apparatus according to claim 1, wherein
the control unit generates the search space that has a linear shape and connects a first point and a second point in the entire search space, the first point and the second point being represented by using the one or several values of the parameters.

3. The optimization apparatus according to claim 1 or 2, wherein
the control unit determines accuracy of the Bayesian model on the basis of a result of comparison between an accuracy evaluation value indicating the accuracy and a threshold, and, in a case where the control unit determines that the accuracy is sufficient, the control unit executes a search for the combination in the entire search space, instead of the search space.

4. An optimization system, comprising:
an optimization apparatus that includes a control unit configured to generate or update, on the basis of input data, a Bayesian model obtained by modeling a problem of searching for a combination of values of a plurality of parameters which gives an optimum value of a characteristic value regarding a target substance, generate, on the basis of one or several input values of parameters among the plurality of parameters, a search space that is included in an entire search space obtained from all combinations of the values of the plurality of parameters and is narrower than the entire search space, and execute a search for the combination using the Bayesian model in the search space; and
a simulation apparatus configured to receive a search result of the combination, calculate the characteristic value on the basis of the search result, and transmit new input data that includes the calculated characteristic value and the search result to the optimization apparatus.

5. An optimization method, comprising
causing a computer to:
generate, on the basis of input data, a Bayesian model obtained by modeling a problem of searching for a combination of values of a plurality of parameters which gives an optimum value of a characteristic value regarding a target substance;
generate, on the basis of one or several input values of parameters among the plurality of parameters, a search space that is included in an entire search space obtained from all combinations of the values of the plurality of parameters and is narrower than the entire search space; and
execute a search for the combination using the Bayesian model in the search space.

6. An optimization program for causing a computer to execute a processing comprising:
generating, on the basis of input data, a Bayesian model obtained by modeling a problem of searching for a combination of values of a plurality of parameters which gives an optimum value of a characteristic value regarding a target substance;
generating, on the basis of one or several input values of parameters among the plurality of parameters, a search space that is included in an entire search space obtained from all combinations of the values of the plurality of parameters and is narrower than the entire search space; and
executing a search for the combination using the Bayesian model in the search space.
